# EUROPÄISCHE PATENTANMELDUNG

(11) **EP 4 506 075 A2**
(43) Veröffentlichungstag der Anmeldung: **12.02.2025**
(21) Anmeldenummer: 24193509.7
(22) Anmeldetag: 08.08.2024
(51) Int. Cl.: B08B 9/032, A61L 2/04

(54) **DAMPFERZEUGUNG IN EINER ANLAGE ZUR BEHANDLUNG VON BEHÄLTERN**

(30) Priorität: 08.08.2023 DE 102023121164
(71) Anmelder: KRONES AG, 93073 Neutraubling (DE)
(72) Erfinder: Soellner, Juergen, 93073 Neutraubling (DE); Gloetzl, Reiner, 93073 Neutraubling (DE); Schafaczek, Bernd, 93073 Neutraubling (DE)
(74) Vertreter: Nordmeyer, Philipp Werner

(57) **Zusammenfassung**

Anlage (1) zur Behandlung von Behältern, vorzugsweise Getränkeabfüllanlage, mit einem Behälterbehandlungsabschnitt (50), der zur Behandlung der Behälter, vorzugsweise zum Befüllen der Behälter mit einem Füllprodukt und/oder Verschließen der Behälter mit je einem Behälterverschluss, eingerichtet ist und in dem bei einer Sterilisierung Kondensat (K) anfällt; einem Schnelldampferzeuger (10) mit einem Dampfkessel (15), der eingerichtet ist, um Speisewasser zu erhitzen, vorzugsweise mittels zumindest eines elektrischen Heizelements (15a), und zu verdampfen, wobei der Schnelldampferzeuger (10) eingerichtet ist, um das im Behälterbehandlungsabschnitt (50) anfallende Kondensat (K) über eine Kondensatleitung (11) zu beziehen und während eines regulären Betriebs in den Dampfkessel (15) als zu verdampfendes Speisewasser einzuleiten.

## Beschreibung

### Technisches Gebiet

Die vorliegende Erfindung betrifft eine Anlage zur Behandlung von Behältern, vorzugsweise eine Getränkeabfüllanlage, sowie ein Verfahren zur Bereitstellung von Dampf in einer solchen Anlage.

### Stand der Technik

Zum Verdampfen flüssiger Medien sind Verdampfer unterschiedlicher Bauart bekannt, die beispielsweise in Getränkeabfüllanlagen dazu verwendet werden, gasförmiges Wasserstoffperoxid zur Desinfektion von produktberührten Anlagenkomponenten bereitzustellen. Die Verdampfer verfügen üblicherweise über eine beheizte Verdampferoberfläche, auf welche das noch flüssige und zu verdampfende Medium aufgesprüht wird und dann verdampft. Die Verdampferoberfläche wird dabei etwa mittels Heißdampf oder mittels elektrischer Heizstäbe geheizt, um die gewünschte Temperatur der Verdampferoberfläche bereitzustellen.

Eine Vorrichtung zum Verdampfen eines flüssigen Mediums in einer Füllproduktabfüllanlage geht beispielsweise aus der WO 2018/060422 A2 hervor.

Die Bereitstellung eines erforderlichen Dampfdrucks erfolgt über einen Dampfkessel, der den produzierten Dampf in ein Dampfnetz einspeist. Der Druck im Dampfnetz ist auf den höchsten Dampfdruck ausgelegt, der von den angeschlossenen Abnehmern gefordert wird. Für alle weiteren Abnehmer wird der Dampfdruck entsprechend reduziert.

Ist ein höherer Druck als der im Dampfnetz vorliegende Druck nur in geringen Mengen erforderlich, so kann a) entweder der Druck im gesamten Dampfnetz erhöht werden, sofern der Dampfkessel für den erforderlichen Druck ausgelegt ist, oder b) ein separater Schnelldampferzeuger für diese Dampfmenge, beispielsweise elektrisch betrieben, eingesetzt werden.

Wird der Dampfdruck gemäß Option a) im gesamten Dampfnetz erhöht, ist es unter Umständen erforderlich, vorhandene Sicherheitseinrichtungen neu auszulegen und gegebenenfalls auszutauschen. Ferner ist der Betrieb des gesamten Dampfnetzes auf einem hohen Druck, der nur für eine geringe Menge an einem Abnehmer erforderlich ist, energetisch nicht sinnvoll. An den übrigen Abnehmern muss der Dampfdruck verlustbehaftet reduziert werden.

Wird gemäß Option b) ein zusätzlicher (elektrischer) Schnelldampferzeuger eingesetzt, kann das vorhandene Dampfnetz auf einem niedrigeren Druckniveau betrieben werden, da die geringe Dampfmenge mit höherem Dampfdruck über einen separaten Dampferzeuger realisiert wird. Zum Speisen der Dampferzeuger wird Wasser beispielsweise aus einer Umkehrosmoseanlage benötigt, um eine wirtschaftliche Laufzeit ohne Abschlämmvorgang zu ermöglichen. Ist die Speisewasserqualität gering und enthält Salz, muss der Dampferzeuger häufig abgeschlämmt werden. Dazu ist es insbesondere bei kleinen Puffervolumen erforderlich, den Dampfdruck zu reduzieren, was zu einem kurzzeitigen Stillstand der Abfüllanlage und einem entsprechenden Verlust des Anlagenwirkungsgrades führt.

### Darstellung der Erfindung

Eine Aufgabe der Erfindung besteht darin, eine verbesserte Anlage zur Behandlung von Behältern sowie ein verbessertes Verfahren zur Bereitstellung von Dampf in einer solchen Anlage vorzuschlagen, insbesondere den Prozess der Dampferzeugung energetisch zu verbessern und/oder zu beschleunigen.

Die Aufgabe wird durch eine Anlage mit den Merkmalen des Anspruchs 1 sowie ein Verfahren mit den Merkmalen des nebengeordneten Verfahrensanspruchs gelöst. Vorteilhafte Weiterbildungen folgen aus den Unteransprüchen, der folgenden Darstellung der Erfindung sowie der Beschreibung bevorzugter Ausführungsbeispiele.

Die Erfindung betrifft eine Anlage zur Behandlung von Behältern mit einem Behälterbehandlungsabschnitt, der zur Behandlung der Behälter eingerichtet ist, sowie einem Schnelldampferzeuger mit einem Dampfkessel, der eingerichtet ist, um Speisewasser zu erhitzen, vorzugsweise mittels zumindest eines elektrischen Heizelements, und zu verdampfen.

Besonders bevorzugt ist die Anlage Teil einer Getränkeabfüllanlage, wobei der Behälterbehandlungsabschnitt in diesem Fall insbesondere eine Füllvorrichtung umfasst, etwa zum Abfüllen von Wasser (still oder karbonisiert), Bier, Saft, Softdrinks, Smoothies, Milchprodukten, Mischgetränken und dergleichen. Der Behälterbehandlungsabschnitt kann eine Füllvorrichtung, Verschließvorrichtung, Streckblassvorrichtung zur Herstellung von Behältern, eine Behälterreinigungsanlage, Etikettierer usw. oder eine Kombination verschiedener Vorrichtungen umfassen, insbesondere eine Füllvorrichtung zum Befüllen von Behältern mit einem Füllprodukt und Verschließen der Behälter mit je einem Behälterverschluss. Die Behälter können Flaschen aus Glas, PET, Dosen, Kartons oder geeignete Behälter anderer Beschaffenheit sein, die mit einem flüssigen Füllproduckt, insbesondere Getränk, befüllt werden können.

In einem solchen Behälterbehandlungsabschnitt kann im Rahmen verschiedener Prozesse Kondensat anfallen, beispielsweise während einer CIP-Behandlung ("Cleaning in Place") oder SIP-Behandlung ("Sterilisation in Place"), bei welcher der Behälterbehandlungsabschnitt ohne wesentliche Demontage auf den produktberührten Flächen gereinigt und/oder sterilisiert und/oder desinfiziert wird. Um eine Sterilisierung zu erreichen, werden erforderliche Medien, beispielsweise Druckluft, über mit Heißdampf beaufschlagte Wärmetauscher erhitzt. Dabei schlägt sich Wasser an Oberflächen der Wärmetauscher im Behälterbehandlungsabschnitt nieder, sobald der Dampf unter den Taupunkt abgekühlt wird. Auf diese Weise entsteht bei der Sterilisierung Kondensat im Behandlungsabschnitt.

Der Schnelldampferzeuger gemäß der Erfindung ist nun eingerichtet, um das im Behälterbehandlungsabschnitt während der Sterilisation anfallende Kondensat über eine Kondensatleitung zu beziehen und in den Dampfkessel als zu verdampfendes Speisewasser einzuleiten.

Der Schnelldampferzeuger wird somit zumindest teilweise, vorzugsweise ausschließlich, mit Kondensat betrieben. Das führt dazu, dass keine separate Wasseraufbereitung erforderlich ist oder eine etwaige Wasseraufbereitung weniger aufwändig realisierbar ist, da das Kondensat bereits über einen sehr geringen Mineralgehalt verfügt. Das Kondensat weist ferner bereits eine hohe Temperatur auf, beispielsweise ca. 90°C, wodurch im Vergleich zu kaltem Speisewasser Energie eingespart sowie die Dampferzeugung beschleunigt werden kann.

Der Schnelldampferzeuger weist vorzugsweise ein Vorlaufgefäß auf, das eingerichtet ist, um das Kondensat über die Kondensatleitung zu beziehen, zu sammeln sowie an den Dampfkessel abzugeben. Das Kondensat im Vorlaufgefäß kann bedarfsweise oder kontinuierlich dem Dampfkessel zugeführt werden. Zu diesem Zweck ist vorzugsweise eine Druckerhöhungspumpe vorgesehen, die eingerichtet ist, um das Kondensat aus dem Vorlaufgefäß in den Dampfkessel zu fördern.

Das im Vorlaufgefäß gesammelte Kondensat kann vorzugsweise über eine Ablaufverrohrung abgeleitet werden, sofern erforderlich.

Der im Schnelldampferzeuger hergestellte Dampf kann in unterschiedlichen Prozessen verwendet werden, insbesondere zum Betrieb eines Verdampfers, der beispielsweise zur Herstellung von gasförmigem Wasserstoffperoxid zur Desinfektion von zu befüllenden Behältern oder Behälterverschlüssen eingerichtet ist.

Vorzugsweise weist die Anlage somit einen Verdampfer auf, der eingerichtet ist, um Dampf aus dem Dampfkessel des Schnelldampferzeugers zu beziehen und ein Behandlungsmittel, vorzugsweise Wasserstoffperoxid, zu verdampfen. Der Schnelldampferzeuger lässt sich auf diese Weise mit geringem maschinenbaulichen Aufwand in eine bestehende Anlage integrieren.

Vorzugsweise weist die Anlage einen Betriebsdampferzeuger auf, der sich vom Schnelldampferzeuger unterscheidet und eingerichtet ist, um Betriebsdampf zu erzeugen. Insbesondere kann der Behälterbehandlungsabschnitt vom Betriebsdampferzeuger mit Betriebsdampf versorgt werden, beispielsweise für eine Sterilisierung während der CIP- und/oder SIP-Behandlung, bei welcher der Behälterbehandlungsabschnitt ohne wesentliche Demontage auf den produktberührten Flächen gereinigt und/oder sterilisiert und/oder desinfiziert wird. Der Betriebsdampf kann alternative oder zusätzliche Funktionen im Behälterbehandlungsabschnitt übernehmen, beispielsweise zum Erhitzen bestimmter Anlagenteile.

Vorzugsweise sind die Temperatur, beispielsweise ca. 160°C, und/oder der Druck, beispielsweise ca. 6 bar, des vom Betriebsdampferzeuger hergestellten Dampfs geringer als die Temperatur, beispielsweise ca. 180°C, und/oder der Druck, beispielsweise ca. 9 bar, des vom Schnelldampferzeuger hergestellten Dampfs. Der Schnelldampferzeuger kann in diesem Fall als "Booster" für den Betriebsdampferzeuger fungieren, wodurch sich bestimmte Prozesse, beispielsweise eine Sterilisation von Behälterverschlüssen, besonders energie- und ressourcenschonend durchführen lassen, da nicht das gesamte Dampfnetz mit Dampf eines höheren Drucks und einer höheren Temperatur versorgt werden muss.

Der Schnelldampferzeuger weist vorzugsweise eine geringere Kapazität als der Betriebsdampferzeuger auf. So ist der Schnelldampferzeuger etwa ausgelegt, um nur einen Bruchteil des Dampfes des Betriebsdampferzeugers herzustellen, beispielsweise ein Hundertstel der Menge des Betriebsdampferzeugers.

Vorzugsweise ist der Betriebsdampferzeuger Teil eines Reinigungskreises, der eingerichtet ist, um den Behälterbehandlungsabschnitt im Rahmen einer CIP-Behandlung zu reinigen und/oder zu sterilisieren und/oder zu desinfizieren. Das im Schnelldampferzeuger während des regulären Betriebs genutzte Kondensat fällt vorzugsweise während der CIP und/oder SIP-Behandlung beim Sterilisieren an.

Vorzugsweise ist der Schnelldampferzeuger über einen CIP-Vorlauf an den Reinigungskreis der Anlage angebunden. Der Anschluss an einen vorhandenen Reinigungszyklus, vorzugsweise Säure-Reinigungszyklus, führt zu längeren Standzeiten, ohne dass ein entsprechender Abschlämmvorgang erforderlich ist. Eventuell anfallende mineralische Rückstände an den Heizelementen können beim nächsten Reinigungszyklus des Behälterbehandlungsabschnitts automatisch mit abgereinigt werden.

Vorzugsweise erfolgt der Anschluss an den Reinigungskreislauf über eine Block&Bleed-Ventilkombination, um zu verhindern, dass es zur Vermischung von Medien bei einer etwaigen Leckage eines Ventils kommt.

Die oben genannte Aufgabe wird ferner durch ein Verfahren zur Bereitstellung von Dampf in einer Anlage zur Behandlung von Behältern, vorzugsweise in einer Getränkeabfüllanlage, gelöst: Das Verfahren weist auf: Erzeugen von Kondensat in einem Behälterbehandlungsabschnitt während einer Sterilisation, wobei der Behälterbehandlungsabschnitt zur Behandlung der Behälter, vorzugsweise zum Befüllen der Behälter mit einem Füllprodukt und/oder Verschließen der Behälter mit je einem Behälterverschluss, eingerichtet ist; während eines regulären Betriebs Einleiten des Kondensats über eine Kondensatleitung in einen Dampfkessel eines Schnelldampferzeugers; Erhitzen und Verdampfen des Kondensats als Speisewasser im Dampfkessel, vorzugsweise mittels zumindest eines elektrischen Heizelements.

Die Merkmale, technischen Wirkungen, Vorteile sowie Ausführungsbeispiele, die in Bezug auf die Anlage beschrieben wurden, gelten analog für das Verfahren.

So weist der Schnelldampferzeuger aus den oben genannten Gründen vorzugsweise ein Vorlaufgefäß auf, welches das Kondensat über die Kondensatleitung bezieht, sammelt sowie an den Dampfkessel abgibt. Das Kondensat wird vorzugsweise mittels einer Druckerhöhungspumpe aus dem Vorlaufgefäß in den Dampfkessel gefördert.

Aus den oben genannten Gründen betreibt der im Dampfkessel erzeugte Dampf vorzugsweise einen Verdampfer, der ein Behandlungsmittel, vorzugsweise Wasserstoffperoxid, verdampft, wobei der Verdampfer das verdampfte Behandlungsmittel vorzugsweise zur Sterilisierung von zu befüllenden Behältern und Behälterverschlüssen während des regulären Betriebs bereitstellt. Vorzugsweise stellt aus den oben genannten Gründen ein Betriebsdampferzeuger, der sich vom Schnelldampferzeuger unterscheidet, Betriebsdampf her, dessen Temperatur und/oder Druck vorzugsweise geringer sind als die Temperatur und/oder der Druck des vom Schnelldampferzeuger hergestellten Dampfs.

Vorzugsweise ist der Betriebsdampferzeuger Teil eines Reinigungskreises, der den Behälterbehandlungsabschnitt im Rahmen einer CIP-Behandlung reinigt und/oder sterilisiert und/oder desinfiziert.

Vorzugsweise fällt während der CIP-Behandlung das Kondensat im Behälterbehandlungsabschnitt an, wobei das Kondensat im Vorlaufgefäß des Schnelldampferzeugers bis zu einer späteren Verwendung gesammelt wird. Somit wird das Kondensat während der Anlagenreinigung aufgefangen und zu einem späteren Zeitpunkt verwendet. Zwischen dem Auffangen des Kondensats und der Verwendung des Kondensats können beispielsweise mehrere Stunden liegen.

Es sei hierin zwischen einem regulären Betrieb, d.h. der eigentlichen Produktion in der Anlage, in der Getränke in Behälter abgefüllt werden, und einem vorbereitenden Reinigungsbetrieb, in dem die Anlage, insbesondere der Behälterbehandlungsabschnitt, einer CIP- und/oder SIP-Behandlung zur Sterilisierung unterzogen wird, unterschieden.

Das Kondensat fällt während des Reinigungsbetriebs des Behälterbehandlungsabschnitts an und wird anschließend im Vorlaufbehälter gesammelt. Ausgehend davon wird das im Vorlaufgefäß gesammelte Kondensat vorzugsweise während des regulären Betriebs des Behälterbehandlungsabschnitts verwendet. Genauer gesagt, das im Vorlaufgefäß gesammelte Kondensat wird vorzugsweise während des regulären Betriebs des Behälterbehandlungsabschnitts dem Schnelldampferzeuger zugeführt, wobei der im Schnelldampferzeuger erzeugte Dampf dann zur Verdampfung des Behandlungsmittels, vorzugsweise Wasserstoffperoxid, verwendet und das verdampfte Behandlungsmittel dem Behälterbehandlungsabschnitt während des regulären Betriebs zugeführt wird.

Das während des regulären Betriebs bei der Verdampfung des Behandlungsmittels anfallende Kondensat, das durch die Abkühlung des vom Schnelldampferzeuger erzeugten Dampfes am Verdampfer für das Behandlungsmittel anfällt, wird ebenfalls dem Vorlaufgefäß des Schnelldampferzeugers zugeführt, so dass sich im regulären Betrieb ein Kreislauf zum Beheizen des Verdampfers ergibt.

Vorzugsweise wird das so hergestellte Behandlungsmittel zur Sterilisation von Behältern und/oder Behälterverschlüssen im Behälterbehandlungsabschnitt verwendet.

Weitere Vorteile und Merkmale der vorliegenden Erfindung sind aus der folgenden Beschreibung bevorzugter Ausführungsbeispiele ersichtlich. Die darin beschriebenen Merkmale können alleinstehend oder in Kombination mit einem oder mehreren der oben dargelegten Merkmale umgesetzt werden, insofern sich die Merkmale nicht widersprechen. Die folgende Beschreibung bevorzugter Ausführungsbeispiele erfolgt dabei mit Bezug auf die begleitende Zeichnung.

### Kurze Beschreibung der Figur

Bevorzugte weitere Ausführungsformen der Erfindung werden durch die nachfolgende Beschreibung der Figur näher erläutert. Dabei zeigt:

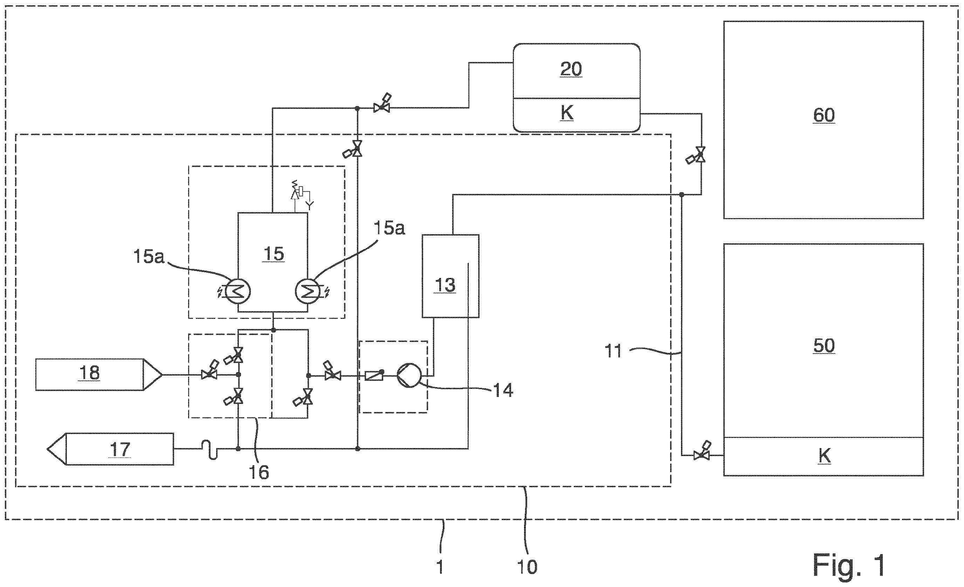

- Figur 1: schematisch eine Anlage zur Behandlung von Behältern mit einem Behälterbehandlungsabschnitt und einem Schnelldampferzeuger.

### Detaillierte Beschreibung bevorzugter Ausführungsbeispiele

Im Folgenden werden bevorzugte Ausführungsbeispiele anhand der Figur 1 beschrieben, die schematisch eine Anlage 1 zur Behandlung von Behältern zeigt. Die Anlage 1 umfasst einen Schnelldampferzeuger 10 und einen Behälterbehandlungsabschnitt 50.

Besonders bevorzugt ist die Anlage 1 eine Getränkeabfüllanlage, wobei der Behälterbehandlungsabschnitt 50 in diesem Fall insbesondere eine Füllvorrichtung umfasst, zum Abfüllen von Wasser (still oder karbonisiert), Bier, Saft, Softdrinks, Smoothies, Milchprodukten, Mischgetränken und dergleichen. Der Behälterbehandlungsabschnitt 50 kann eine Füllvorrichtung, Verschließvorrichtung, Streckblassvorrichtung zur Herstellung von Behältern, eine Behälterreinigungsanlage, Etikettierer usw. oder eine Kombination verschiedener Vorrichtungen umfassen, insbesondere eine Füllvorrichtung zum Befüllen von Behältern mit einem Füllprodukt und Verschließen der Behälter mit je einem Behälterverschluss. Die Behälter können Flaschen aus Glas, PET, Dosen, Kartons oder geeignete Behälter anderer Beschaffenheit sein, die mit einem flüssigen Füllproduckt, insbesondere Getränk, befüllt werden können.

Der Behälterbehandlungsabschnitt 50 kann von einem Betriebsdampferzeuger 60 mit Betriebsdampf versorgt werden, beispielsweise für eine CIP-Behandlung ("Cleaning in Place") oder SIP-Behandlung ("Sterilisation in Place"), bei welcher der Behälterbehandlungsabschnitt 50 ohne wesentliche Demontage auf den produktberührten Flächen gereinigt und/oder sterilisiert und/oder desinfiziert wird. Der Betriebsdampf kann alternative oder zusätzliche Funktionen im Behälterbehandlungsabschnitt 50 übernehmen, beispielsweise zum Erhitzen bestimmter Anlagenteile.

Üblicherweise wird der Betriebsdampf mit vergleichsweise geringer Temperatur, beispielsweise ca. 160°C, und vergleichsweise geringem Druck, beispielsweise ca. 6 bar, angewendet.

Der Schnelldampferzeuger 10 kann bezüglich des Behälterbehandlungsabschnitts 50 extern konzipiert oder teilweise oder vollständig im Behälterbehandlungsabschnitt 50 integriert sein, wodurch er mit bereits vorhandenen Medien versorgt werden kann. Vorzugsweise ist der Schnelldampferzeuger 10 steuerungstechnisch in den Behälterbehandlungsabschnitt 50, insbesondere der Abfüllanlage, eingebunden.

Als Speisewasser für den Schnelldampferzeuger 10 wird anfallendes Kondensat K aus Prozessen im Behälterbehandlungsabschnitt 50 verwendet, das vorzugsweise aus dem Dampfnetz des Betriebsdampferzeugers 60, d.h. insbesondere aus dem Dampfnetz mit niedrigerem Druck, versorgt wird. Zu diesem Zweck wird Kondensat K aus dem Behälterbehandlungsabschnitt 50 über eine Kondensatleitung 11 dem Schnelldampferzeuger 10 zugeführt.

Das über die Kondensatleitung 11 bezogene Kondensat Kwird einem Vorlaufgefäß 13 zugeführt, in dem es aufgefangen und gesammelt wird. Diese Zuführung des Kondensats zum Vorlaufgefäß 13 findet üblicherweise statt, während die das Kondensat K bei der Sterilisierung im Behälterbehandlungsabschnitt 50 anfällt.

Um das Kondensat dann aus dem Vorlaufgefäß 13 während des regulären Betriebs der Behälterbehandlungsvorrichtung wird es über eine Druckerhöhungspumpe 14 aus dem Vorlaufgefäß 13 in einen Dampfkessel 15 gefördert. Der Dampfkessel 15 verfügt über Heizelemente 15a, vorzugsweise elektrische Heizelemente, um das Kondensat K im Dampfkessel 15 zu erhitzen. Die Heizelemente 15a werden regelungstechnisch auf den erforderlichen Dampfdruck geregelt. Ferner verfügt der Dampfkessel 15 vorzugsweise über Sicherheitseinrichtungen, um den Dampfkessel 15 den Vorgaben entsprechend abzusichern.

Der so erzeugte Dampf, der vorzugsweise eine höhere Temperatur und einen höheren Druck, beispielsweise 180°C und 9 bar, als der vom Betriebsdampferzeuger 60 hergestellte Dampf aufweist, kann im Rahmen verschiedener Prozesse verwendet werden. Insbesondere kann der Dampf zum Betrieb, insbesondere zur Temperierung, eines Verdampfers 20 genutzt werden, der beispielsweise zur Herstellung von gasförmigem Wasserstoffperoxid zur Desinfektion von Behältern oder Deckeln vor deren eigentlicher Befüllung mit Füllprodukt.

Der Schnelldampferzeuger 10 kann über einen CIP-Vorlauf 18 an einen Reinigungskreis der Anlage 1 bzw. des Behälterbehandlungsabschnitts 50 angebunden sein, wodurch der Schnelldampferzeuger 10 in einen bereits vorhandenen Reinigungszyklus eingebunden werden kann, um so beispielsweise eine Säurereinigung des Schnelldampferzeugers 10 zu ermöglichen. Der Anschluss an den Reinigungskreislauf erfolgt vorzugsweise über eine Block&Bleed-Ventilkombination 16, um zu verhindern, dass es zur Vermischung von Medien bei einer etwaigen Leckage eines Ventils kommt.

Das im Vorlaufgefäß 13 gesammelte Kondensat K kann gegebenenfalls über eine Ablaufverrohrung 17 abgeleitet werden, sofern eine Entleerung des Vorlaufgefäßes erforderlich ist.

Während der oben genannten CIP-Behandlung des Behälterbehandlungsabschnitts 50, insbesondere unter Verwendung des Betriebsdampferzeugers 60, fällt das Kondensat K im Behälterbehandlungsabschnitt 50 an. Das Kondensat K wird dann im Vorlaufgefäß 13 des Schnelldampferzeugers 10 bis zu einer späteren Verwendung gesammelt.

Somit wird das Kondensat K während der Anlagenreinigung aufgefangen und zu einem späteren Zeitpunkt verwendet. Zwischen dem Auffangen des Kondensats K und der Verwendung des Kondensats können beispielsweise mehrere Stunden liegen.

Ausgehend davon wird das im Vorlaufgefäß 13 gesammelte Kondensat K während des regulären Betriebs des Behälterbehandlungsabschnitts 50 verwendet. In diesem Fall wird das im Vorlaufgefäß 13 gesammelte Kondensat K während des regulären Betriebs des Behälterbehandlungsabschnitts 50 dem Dampfkessel 15 zugeführt, wobei der im Dampfkessel 15 erzeugte Dampf dann zur Beheizung des Verdampfers zur Verdampfung des Behandlungsmittels, vorzugsweise Wasserstoffperoxid, verwendet und das verdampfte Behandlungsmittel dem Behälterbehandlungsabschnitt 50 während des regulären Betriebs zugeführt wird.

Das während des regulären Betriebs durch die Abkühlung des Dampfes am Verdampfer anfallende Kondensat K wird dem Vorlaufgefäß 13 wieder zugeführt. Mit anderen Worten kann im regulären Betrieb ein Kreislauf des Kondensats erreicht werden.

Vorzugsweise wird das so hergestellt Behandlungsmittel zur Sterilisation von Behältern und/oder Behälterverschlüssen im Behälterbehandlungsabschnitt 50 verwendet.

Der Schnelldampferzeuger 10 wird zumindest teilweise, vorzugsweise ausschließlich, mit Kondensat K betrieben. Das führt dazu, dass keine separate Wasseraufbereitung erforderlich ist oder eine etwaige Wasseraufbereitung weniger aufwändig realisierbar ist, da das Kondensat K bereits über einen sehr geringen Mineralgehalt verfügt.

Das Kondensat K weist bereits eine hohe Temperatur auf, beispielsweise ca. 90°C, wodurch im Vergleich zu kaltem Speisewasser Energie eingespart sowie die Dampferzeugung beschleunigt werden kann.

Der etwaige Anschluss an einen vorhandenen Reinigungszyklus, vorzugsweise Säure-Reinigungszyklus, führt zu längeren Standzeiten, ohne dass ein entsprechender Abschlämmvorgang erforderlich ist. Eventuell anfallende mineralische Rückstände an den Heizelementen 15a können beim nächsten Reinigungszyklus des Behälterbehandlungsabschnitts 50 automatisch mit abgereinigt werden.

Soweit anwendbar, können alle einzelnen Merkmale, die in den Ausführungsbeispielen dargestellt sind, miteinander kombiniert und/oder ausgetauscht werden, ohne den Bereich der Erfindung zu verlassen.

### Bezugszeichenliste

- 1: Anlage zur Behandlung von Behältern
- 10: Schnelldampferzeuger
- 11: Kondensatleitung
- 13: Vorlaufgefäß
- 14: Druckerhöhungspumpe
- 15: Dampfkessel
- 15a: Heizelement
- 16: Block&Bleed-Ventilkombination
- 17: Ablaufverrohrung
- 18: CIP-Vorlauf
- 20: Verdampfer
- 50: Behälterbehandlungsabschnitt
- 60: Betriebsdampferzeuger

- K: Kondensat

## Patentansprüche

1. Anlage (1) zur Behandlung von Behältern, vorzugsweise Getränkeabfüllanlage, die aufweist:
einen Behälterbehandlungsabschnitt (50), der zur Behandlung der Behälter, vorzugsweise zum Befüllen der Behälter mit einem Füllprodukt und/oder Verschließen der Behälter mit je einem Behälterverschluss, eingerichtet ist und in dem bei einer Sterilisierung Kondensat (K) anfällt;
einen Schnelldampferzeuger (10) mit einem Dampfkessel (15), der eingerichtet ist, um Speisewasser zu erhitzen, vorzugsweise mittels zumindest eines elektrischen Heizelements (15a), und zu verdampfen, wobei
der Schnelldampferzeuger (10) eingerichtet ist, um das im Behälterbehandlungsabschnitt (50) anfallende Kondensat (K) über eine Kondensatleitung (11) zu beziehen und während eines regulären Betriebs in den Dampfkessel (15) als zu verdampfendes Speisewasser einzuleiten.

2. Anlage (1) nach Anspruch 1, **dadurch gekennzeichnet, dass** der Schnelldampferzeuger (10) ein Vorlaufgefäß (13) aufweist, das eingerichtet ist, um das Kondensat (K) über die Kondensatleitung (11) zu beziehen, zu sammeln sowie an den Dampfkessel (15) abzugeben, wobei das Kondensat (K) vorzugsweise mittels einer Druckerhöhungspumpe (14) aus dem Vorlaufgefäß (13) in den Dampfkessel (15) förderbar ist.

3. Anlage (1) nach Anspruch 1 oder 2, **dadurch gekennzeichnet, dass** die Anlage (1) einen Verdampfer (20) aufweist, der eingerichtet ist, um Dampf aus dem Dampfkessel (15) des Schnelldampferzeugers (10) zu beziehen und ein Behandlungsmittel, vorzugsweise Wasserstoffperoxid, zu verdampfen, wobei der Verdampfer (20) vorzugsweise eingerichtet ist, um das verdampfte Behandlungsmittel in den Behälterbehandlungsabschnitt (50) einzuleiten.

4. Anlage (1) nach einem der vorigen Ansprüche, **dadurch gekennzeichnet, dass** die Anlage (1) einen Betriebsdampferzeuger (60) aufweist, der sich vom Schnelldampferzeuger (10) unterscheidet und eingerichtet ist, um Betriebsdampf zu erzeugen, dessen Temperatur und/oder Druck vorzugsweise geringer sind als die Temperatur und/oder der Druck des vom Schnelldampferzeuger (10) hergestellten Dampfs.

5. Anlage (1) nach Anspruch 4, **dadurch gekennzeichnet, dass** der Betriebsdampferzeuger (60) Teil eines Reinigungskreises ist, der eingerichtet ist, um den Behälterbehandlungsabschnitt (50) im Rahmen einer CIP- und/oder SIP-Behandlung zu reinigen und/oder zu sterilisieren und/oder zu desinfizieren, wobei das Kondensat (K) während der CIP- und/oder SIP-Behandlung anfällt.

6. Anlage (1) nach Anspruch 5, **dadurch gekennzeichnet, dass** der Schnelldampferzeuger (10) über einen CIP-Vorlauf (18) an den Reinigungskreis der Anlage (1) angebunden ist, wobei der Anschluss an den Reinigungskreislauf vorzugsweise über eine Block&Bleed-Ventilkombination (16) erfolgt.

7. Verfahren zur Bereitstellung von Dampf in einer Anlage (1) zur Behandlung von Behältern, vorzugsweise in einer Getränkeabfüllanlage, das aufweist:
Erzeugen von Kondensat (K) in einem Behälterbehandlungsabschnitt (50) bei einer Sterilisierung der Behälterbehandlungsabschnitts (50), der zur Behandlung der Behälter, vorzugsweise zum Befüllen der Behälter mit einem Füllprodukt und/oder Verschließen der Behälter mit je einem Behälterverschluss, eingerichtet ist;
Während eines regulären Betriebs Einleiten des Kondensats (K) über eine Kondensatleitung (11) in einen Dampfkessel (15) eines Schnelldampferzeugers (10);
Erhitzen und Verdampfen des Kondensats (K) als Speisewasser im Dampfkessel (15), vorzugsweise mittels zumindest eines elektrischen Heizelements (15a).

8. Verfahren nach Anspruch 7, **dadurch gekennzeichnet, dass** der Schnelldampferzeuger (10) ein Vorlaufgefäß (13) aufweist, welches das Kondensat (K) über die Kondensatleitung (11) bezieht, sammelt sowie an den Dampfkessel (15) abgibt, wobei das Kondensat (K) vorzugsweise mittels einer Druckerhöhungspumpe (14) aus dem Vorlaufgefäß (13) in den Dampfkessel (15) gefördert wird.

9. Verfahren nach Anspruch 7 oder 8, **dadurch gekennzeichnet, dass** der im Dampfkessel (15) erzeugte Dampf einen Verdampfer (20) betreibt, der ein Behandlungsmittel, vorzugsweise Wasserstoffperoxid, verdampft, wobei der Verdampfer (20) das verdampfte Behandlungsmittel zur Behandlung von zu befüllenden Behältern und/oder Behälterverschlüssen bereitstellt.

10. Verfahren nach einem der Ansprüche 7 bis 9, **dadurch gekennzeichnet, dass** ein Betriebsdampferzeuger (60), der sich vom Schnelldampferzeuger (10) unterscheidet, Betriebsdampf herstellt, dessen Temperatur und/oder Druck vorzugsweise geringer sind als die Temperatur und/oder der Druck des vom Schnelldampferzeuger (10) hergestellten Dampfs.

11. Verfahren nach Anspruch 10, **dadurch gekennzeichnet, dass** der Betriebsdampferzeuger (60) Teil eines Reinigungskreises ist, der den Behälterbehandlungsabschnitt (50) im Rahmen einer CIP- und/oder SIP-Behandlung reinigt und/oder sterilisiert und/oder desinfiziert.

12. Verfahren nach Anspruch 8 und 11, **dadurch gekennzeichnet, dass** während der CIP- und/oder SIP-Behandlung des Behälterbehandlungsabschnitts (50) das Kondensat (K) anfällt, wobei das Kondensat (K) im Vorlaufgefäß (13) des Schnelldampferzeugers (10) bis zu einer späteren Verwendung gesammelt wird.

13. Verfahren nach Anspruch 8 und 12, **dadurch gekennzeichnet, dass** das im Vorlaufgefäß (13) gesammelte Kondensat (K) während eines regulären Betriebs des Behälterbehandlungsabschnitts (50) dem Dampfkessel (15) zugeführt wird, wobei der somit im Dampfkessel (15) erzeugte Dampf im Verdampfer (20) zur Verdampfung des Behandlungsmittels, vorzugsweise Wasserstoffperoxid, verwendet wird, und wobei das verdampfte Behandlungsmittel während des regulären Betriebs dem Behälterbehandlungsabschnitt (50) zugeführt wird, vorzugsweise zur Sterilisation von Behältern und/oder Behälterverschlüssen.
